# EUROPEAN PATENT APPLICATION

(11) **EP 1 069 184 A1**
(43) Date of publication of application: **17.01.2001**
(21) Application number: 99202341.6
(22) Date of filing: 16.07.1999
(51) Int. Cl.: C12N 15/12, C07K 14/705, A61K 38/17, C12Q 1/68

(54) **Human anion transporter gene implicated in Salla disease and lysosomal sialic acid transport**

(71) Applicant: Akzo Nobel N.V., 6824 BM Arnhem (NL)
(72) Inventor: The designation of the inventor has not yet been filed
(74) Representative: Hermans, F.G.M.

(57) **Abstract**

This invention decribes a novel human sialic acid transporter. This invention also encompasses nucleic acids encoding this protein. The protein can be used in the identification of activators or inhibitors. Several mutations have been observed in patients suffering from genetic disorders such as lysosomal storage disorders (Salla disease (SD) and the more severe infantile type ISSD). These mutations are informative in a diagnostic context.

SEQUENCE LISTING

<110> AKZO NOBEL N.V.

<120> Novel anion transporter gene

<130> salla

<140>
<141>

<160> 16

<170> PatentIn Ver. 2.1

<210> 1
<211> 495
<212> PRT
<213> Homo sapiens

<400> 1
Met Arg Ser Pro Val Arg Asp Leu Ala Arg Asn Asp Gly Glu Glu Ser
  1               5                  10                  15

Thr Asp Arg Thr Pro Leu Leu Pro Gly Ala Pro Arg Ala Glu Ala Ala
             20                  25                  30

Pro Val Cys Cys Ser Ala Arg Tyr Asn Leu Ala Ile Leu Ala Phe Phe
         35                  40                  45

Gly Phe Phe Ile Val Tyr Ala Leu Arg Val Asn Leu Ser Val Ala Leu
     50                  55                  60

Val Asp Met Val Asp Ser Asn Thr Thr Leu Glu Asp Asn Arg Thr Ser
 65                  70                  75                  80

Lys Ala Cys Pro Glu His Ser Ala Pro Ile Lys Val His His Asn Gln
                 85                  90                  95

Thr Gly Lys Lys Tyr Gln Trp Asp Ala Glu Thr Gln Gly Trp Ile Leu
            100                 105                 110

Gly Ser Phe Phe Tyr Gly Tyr Ile Ile Thr Gln Ile Pro Gly Gly Tyr
        115                 120                 125

Val Ala Ser Lys Ile Gly Gly Lys Met Leu Leu Gly Phe Gly Ile Leu
    130                 135                 140

Gly Thr Ala Val Leu Thr Leu Phe Thr Pro Ile Ala Ala Asp Leu Gly

Ile Ala Pro Ser Tyr Ala Gly Ile Leu Leu Gly Ile Thr Asn Thr Phe
            420                 425                 430

Ala Thr Ile Pro Gly Met Val Gly Pro Val Ile Ala Lys Ser Leu Thr
        435                 440                 445

Pro Asp Asn Thr Val Gly Glu Trp Gln Thr Val Phe Tyr Ile Ala Ala
    450                 455                 460

Ala Ile Asn Val Phe Gly Ala Ile Phe Phe Thr Leu Phe Ala Lys Gly
465                 470                 475                 480

Glu Val Gln Asn Trp Ala Leu Asn Asp His His Gly His Arg His
                485                 490                 495

<210> 2
<211> 2512
<212> DNA
<213> Homo sapiens

<400> 2
cggctacttt gcgccaatcc tacgagaact cccagaactc cgcttcccta gtccaaccca 60
agccagagtt gcccacacct aagatggcgg cggggggcgg agtcggcgcg gccgcctctg 120
ggcgggaccg cggggactag acgtggccgc ggggcggtgt catcgccccc gccccgcccg 180
gtccagccag ctcggcccgg gggcttcggg ctgtcgggcc ggcgctccct tctctgccag 240
gtggcgagta cacctgctca cgtaggcgtc atgaggtctc cggttcgaga cctggcccgg 300
aacgatggcg aggagagcac ggaccgcacg cctcttctac cgggcgcccc acgggccgaa 360
gccgctccag tgtgctgctc tgctcgttac aacttagcaa ttttggcctt ttttggtttc 420
ttcattgtgt atgcattacg tgtgaatctg agtgttgcgt tagtggatat ggtagattca 480
aatacaactt tagaagataa tagaacttcc aaggcgtgtc cagagcattc tgctcccata 540
aaagttcatc ataatcaaac gggtaagaag taccaatggg atgcagaaac tcaaggatgg 600
attctcggtt ccttttttta tggctacatc atcacacaga ttcctggagg atatgttgcc 660
agcaaaatag gggggaaaat gctgctagga tttgggatcc ttggcactgc tgtcctcacc 720
ctgttcactc ccattgctgc agatttagga gttggaccac tcattgtact cagagcacta 780
gaaggactag gagagggtgt tacatttcca gccatgcatg ccatgtggtc ttcttgggct 840
ccccctcttg aaagaagcaa acttcttagc atttcatatg caggagcaca gcttgggaca 900
gtaatttctc ttcctctttc tggaataatt tgctactata tgaattggac ttatgtcttc 960
tacttttttg gtactattgg aatattttgg tttcttttgt ggatctggtt agttagtgac 1020
acaccacaaa aacacaagag aatttcccat tatgaaaagg aatacattct ttcatcatta 1080
agaaatcagc tttcttcaca gaagtcagtg ccgtgggtac ccattttaaa atccctgcca 1140
ctttgggcta tcgtagttgc acacttttct tacaactgga ctttttatac tttattgaca 1200
ttattgccta cttatatgaa ggagatccta aggttcaatg ttcaagagaa tgggttttta 1260
tcttcattgc cttatttagg ctcttggtta tgtatgatcc tgtctggtca agctgctgac 1320
aatttaaggg caaaatggaa tttttcaact ttatgtgttc gcagaatttt tagccttata 1380
ggaatgattg gacctgcagt attcctggta gctgctggct tcattggctg tgattattct 1440
ttggccgttg ctttcctaac tatatcaaca acactgggag gcttttgctc ttctggattt 1500

agcatcaacc atctggatat tgctccttcg tatgctggta tcctcctggg catcacaaat 1560
acatttgcca ctattccagg aatggttggg cccgtcattg ctaaaagtct gacccctgat 1620
aacactgttg gagaatggca aaccgtgttc tatattgctg ctgctattaa tgtttttggt 1680
gccattttct ttacactatt cgccaaaggt gaagtacaaa actgggctct caatgatcac 1740
catggacaca gacactgaag gaaccaataa ataatcctgc ctctattaat gtatttttat 1800
ttatcatgta acctcaaagt gccttctgta ttgtgtaagc attctatgtc tttttttaat 1860
tgtacttgta ttagattttt aaggcctata atcatgaaat atcactagtt gccagaataa 1920
taaaatgaac tgtgtttaat tatgaataat atgtaagcta ggacttctac tttaggttca 1980
catacctgcc tgctagtcgg gcaacatgaa gtaggacagt tctgttgatt ttttagggcc 2040
atactaaagg gaatgagctg aaacagacct cctgatacct ttgcttaatt aaactagatg 2100
ataattctca ggtactgata aacacctgtt gttgttcact ttcctcataa aaattgtcag 2160
ctctctctga cacttagacc tcaaacttta gcatctctgt ggagctgcca tccactgtat 2220
aatttcgcct ggcaactgga ctgaggggag tgtgcccagg cagctgccaa gcactccctc 2280
cctggcttca gggtcagagt gcccagcgtt tatcagaggc agcatccaag cccagagcca 2340
gtgtcgactc ttcggctggt gcctttcctc tgaggggcta tcaatgtgta gataaagccc 2400
tgagtaggca agagcagtga gatccactgc tatggtcttg atacatcctc aaactttccc 2460
ttcccagcac agaggaatat tggctggcat gcaacctgca aaagaaaaat gc 2512

<210> 3
<211> 20
<212> DNA
<213> Homo sapiens

<400> 3
cagtgtgctg ctctgctcgt 20

<210> 4
<211> 20
<212> DNA
<213> Homo sapiens

<400> 4
gtggcgagta cacctgctca 20

<210> 5
<211> 20
<212> DNA
<213> Homo sapiens

<400> 5
gcaatgggag tgaacagggt 20

<210> 6
<211> 21
<212> DNA

<213> Homo sapiens

<400> 6
gatttgggat ccttggcact g                                                21

<210> 7
<211> 20
<212> DNA
<213> Homo sapiens

<400> 7
gtacccacgg cactgacttc                                                  20

<210> 8
<211> 20
<212> DNA
<213> Homo sapiens

<400> 8
ggagcacagc ttgggacagt                                                  20

<210> 9
<211> 20
<212> DNA
<213> Homo sapiens

<400> 9
cagcagcttg accagacagg                                                  20

<210> 10
<211> 21
<212> DNA
<213> Homo sapiens

<400> 10
tgccactttg ggctatcgta g                                                21

<210> 11
<211> 21
<212> DNA
<213> Homo sapiens

<400> 11
gaacacggtt tgccattctt c                                                21

<210> 12
<211> 21
<212> DNA
<213> Homo sapiens

<400> 12
cctcctgggc atcacaaata c                                                21

<210> 13
<211> 22
<212> DNA
<213> Homo sapiens

<400> 13
aacagaactg tcctacttca tg                                               22

<210> 14
<211> 18
<212> DNA
<213> Homo sapiens

<400> 14
cagtgtgctg ctctgctc                                                    18

<210> 15
<211> 18
<212> DNA
<213> Homo sapiens

<400> 15
cagtgtgctg ctctgctt                                                    18

<210> 16
<211> 21
<212> DNA
<213> Homo sapiens

<400> 16
aggccaaaat tgctaagttg t                                                21

145                 150                 155                 160

Val Gly Pro Leu Ile Val Leu Arg Ala Leu Glu Gly Leu Gly Glu Gly
                165                 170                 175

Val Thr Phe Pro Ala Met His Ala Met Trp Ser Ser Trp Ala Pro Pro
            180                 185                 190

Leu Glu Arg Ser Lys Leu Leu Ser Ile Ser Tyr Ala Gly Ala Gln Leu
        195                 200                 205

Gly Thr Val Ile Ser Leu Pro Leu Ser Gly Ile Ile Cys Tyr Tyr Met
    210                 215                 220

Asn Trp Thr Tyr Val Phe Tyr Phe Phe Gly Thr Ile Gly Ile Phe Trp
225                 230                 235                 240

Phe Leu Leu Trp Ile Trp Leu Val Ser Asp Thr Pro Gln Lys His Lys
                245                 250                 255

Arg Ile Ser His Tyr Glu Lys Glu Tyr Ile Leu Ser Ser Leu Arg Asn
            260                 265                 270

Gln Leu Ser Ser Gln Lys Ser Val Pro Trp Val Pro Ile Leu Lys Ser
        275                 280                 285

Leu Pro Leu Trp Ala Ile Val Val Ala His Phe Ser Tyr Asn Trp Thr
    290                 295                 300

Phe Tyr Thr Leu Leu Thr Leu Leu Pro Thr Tyr Met Lys Glu Ile Leu
305                 310                 315                 320

Arg Phe Asn Val Gln Glu Asn Gly Phe Leu Ser Ser Leu Pro Tyr Leu
                325                 330                 335

Gly Ser Trp Leu Cys Met Ile Leu Ser Gly Gln Ala Ala Asp Asn Leu
            340                 345                 350

Arg Ala Lys Trp Asn Phe Ser Thr Leu Cys Val Arg Arg Ile Phe Ser
        355                 360                 365

Leu Ile Gly Met Ile Gly Pro Ala Val Phe Leu Val Ala Ala Gly Phe
    370                 375                 380

Ile Gly Cys Asp Tyr Ser Leu Ala Val Ala Phe Leu Thr Ile Ser Thr
385                 390                 395                 400

Thr Leu Gly Gly Phe Cys Ser Ser Gly Phe Ser Ile Asn His Leu Asp
                405                 410                 415

## Description

The invention relates to a polynucleotide encoding a novel anion transporter protein, the protein encoded by that polynucleotide, a recombinant cell expressing this protein as well as a method for identification compounds effecting the transporter function of the protein. The invention furthermore relates to a method for identifying polymorphisms as well as to nucleotide acid segments used in that method.

Sialic acid storage diseases (SASD) are characterized by accumulation of the acid monosaccharide sialic acid in the lysosomal compartment in several tissues and cell types (Gahl, W.A. et al. in The Metabolic and Molecular Bases of Inherited Disease. (eds Scriver, C.R., Beaudet, A.L., Sly, W.S. & Valle, D., 7th Ed.) 3763-3797 (McGraw-Hill, New York, 1995)). Two distinct phenotypes of SASD can be distinguished; the originally described adult type, Salla disease and the more severe infantile type ISSD. Salla disease is prevalent in the Finnish population but occasional patients have been found worldwide. Clinically Salla disease patients are normal at birth, present signs of hypotonia and ataxia during the first six months of life and thereafter show delayed motor and intellectual development. Dysmorphic features as well as visceral manifestations are absent and the life span of patients is close to normal. Developmental delay is associated with disturbed myelination in the central nervous system as seen with MRI. Clinical presentation of ISSD is severe leading to death at early age. Intrauterine manifestations, such as fetal hydrops and ascites have been reported and failure to thrive, hepatosplenomegaly, coarse facial features, mild bone dysplasia and hypopigmented skin are frequently encountered in ISSD. Rare intermediate phenotypes have also been described. Enlarged lysosomes can be seen in skin, liver, bone marrow and several other tissues in EM study, evidencing the lysosomal accumulation of free sialic acid. Urinary excretion of free sialic acid is approximately 10-fold in Salla disease and 100-fold in ISSD allowing an easy screening test for these disorders.

Early studies in which cultured fibroblasts were loaded with sialic acid demonstrated retention of sialic acid in mutant cells providing indirect evidence for a defective transport system as primary cause of sialic acid storage disorders. From purified lysosomal membranes of rat liver a novel carrier for a wide range of structurally unrelated acid monosaccharides including sialic acid, glucuronic acid and glucoaldonic acids was characterized (Mancini et al. J. Biol. Chem. 264, 15247-15254 (1989)). By testing the transport in lysosomal membranes purified from cultured fibroblasts it was observed that transport of sialic acid and glucuronic acid is defective in patients with Salla disease and ISSD. EBV-transformed lymphoblasts from parents of Salla disease patients had intermediate transport rates suggesting gene-dosage in obligate carriers of an autosomal recessive disorder.

The gene for Salla disease was assigned to chromosome 6q14-q15 in linkage study with Finnish family material (Schleutker, J. et al. Genomics 27, 286-292 (1995)). Based on allelic association and haplotype analysis the critical area harboring the Salla disease gene was restricted to approximately 200 kb. The common haplotype by allele association of the two closest markers (AFMa336zf9, AFMb281wg9) was present in 91 percent of the parental Salla disease chromosomes, but only in one non-Salla disease chromosome strongly suggesting a founder mutation in the Finnish population (Schleutker, J. et al. J. Med. Genet. 33, 36-41 (1996)). Salla disease and ISSD were further shown to be allelic disorders using linkage analysis in ISSD families from various ethnic origin. A physical map was constructed to cover the 200 kb area flanked by the loci D6S280 and D6S1622 providing the basis for precise physical positioning of the gene (Leppanen, P. et al. Genomics 37, 62-67 (1996)).

Purification of the lysosomal sialic acid transporter from rat liver revealed that a single 57 kDa hydrophobic polypeptide accounts for transport of both acid monosaccharides and aliphatic (non-sugar) monocarboxylates.

According to the present invention a new human ACS gene mapping to the critical SASD region in the immediate vicinity of the locus D6S1596 has been identified. Mutations which predictively disrupt the transport function were found in both Salla disease and ISSD patients indicating that this ACS gene codes for the lysosomal membrane sialic acid transporter and that the mutations in the gene arc the primary cause of lysosomal sialic acid storage diseases.

The major type of sialic acid in humans is N-acetylneuraminic acid (NeuAc). In other mammals, a variable but often minor percentage of the sialic acids is represented by N-glycolylneuraminic acid (NeuGc). These monocarboxylic N-substituted monosaccharides are essential components of oligosaccharides (OGS) either present in glycoproteins or gangliosides. OGS-bound sialic acids are involved in main metabolic functions like immunological processes, hormonal responses, signal transmission in neurons, tumor progression, cell adhesion and protection from premature degradation.

The expression pattern of genes encoding lysosomal enzymes reflects their ubiquitous distribution in different animal tissues and cell types that in turn relates to the relative amount and quality of substrates to be degraded. In this respect these genes can be compared with housekeeping genes. However, this does not imply that their expression is not regulated, for instance, at certain stages of embryonal development, tissue differentiation or under specific physiological conditions.

In recent years unexpected roles for lysosomal enzymes have emerged or have been postulated. A model of T and B lymphocytes interaction proposes that resting splenic B cells do not interact with a specific T cell population unless they are activated and do present antigens to T helper cells.

This interaction process is mediated by membrane bound glycoprotein(s) on the surface of B cells that should be devoid of terminal sialic acid residues. The lysosomal enzymes have been suggested to play a role in specific degradation or regulation of bioactive peptides including oxcytocin, substance P, angiotensin I and endothelin. Finally, an important role is known to be played by certain lysosomal enzymes in reproductive processes, like ovulation, acrosome reaction and egg implantation.

The degradative activity of lymphocytes play an important role in a number of essential immune functions. It is well known that synthesis of the enzymes and structural components of the lysosomal compartment play an important role in lymphocyte activation. A model of T- and B-lymphocytes interaction proposes that resting splenic B-cells do not interact with a specific T-cell population unless they are activated and do present antigens to T-helper cells. This interaction process is mediated by membrane bound glycoproteins(s) on the surface of B-cells that should be devoid of terminal sialic acid residues.

It has been hypothesized that lysosomal enzymes involved in desialylation could be involved in this proces (Kearse et al., Cell Immunol 115: 334-351 (1988), J. Immunol 140: 1770-1778 (1988)).

The gene according to the invention encodes an inorganic phosphate cotransporter family member protein belonging to the anion cation symporter family (ACS). This family contains several eukaryotic, including mammalian inorganic transporters (Na+/phosphate cotransporters) as well as prokaryotic organic anion transporters (among which proton acid sugar symporters for hexuronate and glucarate). ACS members transport their substrates in symport with either Na+ or H+, depending on the system. The ACS family belongs to the extensive group of the major facilitator superfamily (MFS) of transporters. The MFS is one of the two largest families of membrane transporters found on earth. It is present ubiquitously in bacteria, archaea, and eukarya and includes members that can function by solute uniport, solute/cation symport, solute/cation antiport and or solute/solute antiport with inwardly and or outwardly directed polarity. All homologous proteins known thusfar were identified on the basis of sequence similarity. Phylogenetic analysis revealed the occurrence of 17 distinct families within this group of proteins, each of which generally transports a single class of compounds. Compounds transported by these permease family members (hereinafter called targets) include simple sugars, oligosaccharides, inositols, drugs, aminoacids, nucleosides, organophosphate esters, krebs cycle metabolites, and a large variety of organic and inorganic anions and cations. The sialin transporter shows extensive homology over the whole open reading frame with rat and human brain sodium phosphate symporters and with a proton hexuronate symporter. All permeases of the MFS possess either 12 or 14 putative or established transmembrane alpha-helical spanners. A human brain specific sodium-dependent inorganic phosphate cotransporter (BNPI) has been described by Bellocchio et al. J. of Neuroscience 18, 8648-8659 (1998). This Na+ dependent inorganic phosphate transporter localizes almost exclusively to terminals forming asymetric excitatory-type synapses suggesting a role in multiple glutamatergic neurotransmission pathways. Loss of function mutations in the C. elegans homologous gene (EAT-4) causes defective glutamatergic chemical transmission and appears to have little effect on other functions of neurons (Lee et al. J. Neuroscience 19, 159-167 (1999)).

A better knowledge of these transporters, their mechanism of action and of the ligands which are transported by these proteins might help to create a better insight in normal physiology and pathology, which eventually will lead to better treatment of sialic acid associated diseases and abnormalities linked to suboptimal Sialin transporter functioning.

The present invention provides for such a novel transporter. More specific, the present invention provides for a polynucleotide sequence comprising encoding SEQ ID NO: 1.

The complete genetic sequence can be used in the preparation of vector molecules for expression of the protein in suitable host cells. Complete genes or variants thereof can be derived from cDNA or genomic DNA from natural sources or synthesized using known methods.

The invention also includes the entire mRNA sequence part of which is indicated in SEQ ID NO: 2. A complete coding DNA sequence is shown in SEQ ID NO:2 nucleotides 271-1755. Furthermore, to accommodate codon variability, the invention also includes sequences coding for the same amino acid sequences as the sequences disclosed herein. Also portions of the coding sequences coding for individual domains of the expressed protein are part of the invention as well as allelic and species variations thereof. Sometimes, a gene is expressed in a certain tissue as a splicing variant, resulting in an altered 5' or 3' mRNA or the inclusion of an additional exon sequence. These sequences as well as the proteins encoded by these sequences all are expected to perform the same or similar functions and form also part of the invention.

The sequence information as provided herein should not be so narrowly construed as to require inclusion of erroneously identified bases. The specific sequence disclosed herein can be readily used to isolate the complete genes which in turn can easily be subjected to further sequence analyses thereby identifying sequencing errors.

Thus, in one aspect, the present invention provides for isolated polynucleotides encoding a novel transporter.

The DNA according to the invention may be obtained from cDNA. The tissues preferably are from human origin. Preferably ribonucleic acids are isolated from fetal brain, fetal liver, fetal spleen, placenta or other tissues. Alternatively, the coding sequence might be genomic DNA, or prepared using DNA synthesis techniques. The polynucleotide may also be in the form of RNA. If the polynucleotide is DNA, it may be in single stranded or double stranded form. The single strand might be the coding strand or the non-coding (anti-sense) strand.

The present invention further relates to polynucleotides having slight variations or have polymorphic sites. Polynucleotides having slight variations encode polypeptides which retain the same biological function or activity as the natural, mature protein. Alternatively, also fragments of the above mentioned polynucleotides which code for domains of the transporter proteins which still are capable of binding to targets are embodied in the invention. Polymorpic sites arc useful for diagnostic purposes.

Such polynucleotides can be identified by hybridization under preferably highly stringent conditions. According to the present invention the term "stringent" means washing conditions of 1 x SSC, 0.1% SDS at a temperature of 65 °C; highly stringent conditions refer to a reduction in SSC towards 0.3 x SSC.

The DNA according to the invention will be very useful for *in vivo* or *in vitro* expression of the novel transporter proteins according to the invention in sufficient quantities and in substantially pure form.

Northern blot analysis of human RNA samples showed two major transcripts (4.5 and 3.5 kb) which are ubiquitously expressed in heart, kidney, skeletal muscle, liver, placenta and brain. The transporter according to the invention furthermore distinguishes itself from the other known major facilitator transport family members in differences in tissue distribution, indicating that there may be important differences between these tissues at the level of signal transduction.

The identification of a novel ACS family transporter could be a major step forward to the existing clinical therapies based on the lack of the gene product responsible for abnormalities and/or diseases which are ascribed to these transporters. The transporter according to the invention will be useful in the development of target analogues that selectively activate or block this transporter according to the invention. This should be considered as one of the major advantages of the present invention.

Thus, the invention will facilitate better treatment of sialic acid associated diseases and/or the design of new drugs for CNS and hemic immune related disorders, in which sialic acid and/or sugars may play an important role.

Also functional equivalents that is transporters comprising SEQ ID NO: 1 or parts thereof having variations of the sequence while still maintaining functional characteristics, are included in the invention.

The variations that can occur in a sequence may be demonstrated by (an) amino acid difference(s) in the overall sequence or by deletions, substitutions, insertions, inversions or additions of (an) amino acid(s) in said sequence. Amino acid substitutions that are expected not to essentially alter biological and immunological activities, have been described. Amino acid replacements between related amino acids or replacements which have occurred frequently in evolution are, inter alia Ser/Ala, Ser/Gly, Asp/Gly, Asp/Asn, Ile/Val (see Dayhof, M.D., Atlas of protein sequence and structure, Nat. Biomed. Res. Found., Washington D.C., 1978, vol. 5, suppl. 3). Based on this information Lipman and Pearson developed a method for rapid and sensitive protein comparison (Science, 1985, 227, 1435-1441) and determining the functional similarity between homologous polypeptides. It will be clear that also polynucleotides coding for such variants are part of the invention.

The polypeptides according to the present invention include the polypeptides comprising SEQ ID NO:1 but also polypeptides with a similarity of 70%, preferably 90%, more preferably 95%. Also portions of such polypeptides still capable of conferring biological effects are included. Especially portions which still bind to targets form part of the invention. Such portions may be functional per se, e.g. in solubilized form or they might be linked to other polypeptides, either by known biotechnological ways or by chemical synthesis, to obtain chimeric proteins. Such proteins might be useful as therapeutic agent by preventing the target from interacting with the natural transporters in the body.

Alternatively, downregulation of the expression level of the transporter can be obtained by using anti-sense nucleic acids through triple-helix formation (Cooney et al., 1988, Science, 241, 456-459) or by binding to the mRNA. This in itself could also lead to treatment of sialic acid related diseases or lysosomal storage disorders in general.

A wide variety of host cell and cloning vehicle combinations may be usefully employed in cloning the nucleic acid sequence coding for the transporter or the target-binding domain of the invention. For example, useful cloning vehicles may include chromosomal, non-chromosomal and synthetic DNA sequences such as various known bacterial plasmids and wider host range plasmids and vectors derived from combinations of plasmids and phage or virus DNA.

Vehicles for use in expression of the transporter or target-binding domain thereof of the present invention will further comprise control sequences operably linked to the nucleic acid sequence coding for the target-binding domain. Such control sequences generally comprise a promoter sequence and sequences, which regulate and/or enhance expression levels. Of course control and other sequences can vary depending on the host cell selected.

Suitable expression vectors are for example bacterial or yeast plasmids, wide host range plasmids and vectors derived from combinations of plasmid and phage or virus DNA. Vectors derived from chromosomal DNA are also included. Furthermore an origin of replication and/or a dominant selection marker can be present in the vector according to the invention. The vectors according to the invention are suitable for transforming a host cell.

Recombinant expression vectors comprising the DNA of the invention as well as cells transformed with said DNA or said expression vector also form part of the present invention.

Suitable host cells according to the invention are bacterial host cells, yeast and other fungi, plant or animal host such as Chinese Harnster Ovary cells or monkey cells. Thus, a host cell which comprises the DNA or expression vector according to the invention is also within the scope of the invention. The engineered host cells can be cultured in conventional nutrient media which can be modified e.g. for appropriate selection, amplification or induction of transcription. The culture conditions such as temperature, pH, nutrients etc. are well known to those ordinary skilled in the art.

The techniques for the preparation of the DNA or the vector according to the invention as well as the transformation or transfection of a host cell with said DNA or vector are standard and well known in the art, see for instance Sambrook et al., Molecular Cloning: A laboratory Manual. 2nd Ed., Cold Spring Harbor Laboratory, Cold Spring Harbor, NY, 1989.

The protein according to the invention can be recovered and purified from recombinant cell cultures by common biochemical purification methods (as decribed in Havelaar et al, J. Biol. Chem. 273, 34568-34574 (1998)) including ammonium sulfate precipitation, extraction, chromatography such as hydrophobic interaction chromatography, cation or anion exchange chromatography or affinity chromatography and high performance liquid chromatography. If necessary, also protein refolding steps can be included. Alternatively the protein can be expresssed and purified as a fusion protein containing ("tags") which can be used for affinity purification.

Sialic acid transport proteins according to the present invention can be used for the *in vitro* or *in vitro* identification of novel targets or analogues. For this purpose e.g. binding studies can be performed with cells transformed with DNA according to the invention or an expression vector comprising DNA according to the invention, said cells expressing the transporter according to the invention.

Alternatively also the novel purified transporter according to the invention as well as the target-binding domain thereof can be used in an assay for the identification of functional targets or analogues for the transporter.

Methods to determine target binding as well as *in vitro* and *in vivo* assays to determine biological transport activity of transporters are well known. In general, transporter proteins shuttle compounds across membranes. This feature can be used ideally for testing binding, stimulation or inhibition of a functional response.

The transporter can be produced in high quantities by expression of the cDNA constructs described above. After purification of the expressed protein, this transporter can be reconstituted in an artificial membrane to measure its transport function. The transporter is a very hydrophobic integral membrane protein that can be solubilized with the help of detergents like triton X-100. In this state the protein can be purified (if necessary). Subsequently the micelles of protein and detergents will be mixed with purified egg yolk phospholipids. The now formed mixed micelles of protein-detergent-phospholipids will be added to Amberlite XAD-2(Fluka inc.) beads in a ratio of 1:1, followed by 30 minutes rotation at room temperature. In this manner the detergent concentration will be lowered slowly and proteoliposomes will be formed with the sialin transporter incorporated in their membranes. Proteoliposomes can be separated from beads and bound detergent by a short centrifugation step. The resulting proteoliposomes can be used for transport assays. Uptake of e.g. radioactive sialic and or glucuronic acid can be measured as described earlier for transport studies in lysosomal membrane vesicles (see Mancini et al. J. Biol. Chem. 264, 15247-15254 (1989)). Using such an assay compounds can be identified that influence the uptake of sialic and glucuronic acid. Compounds to be tested will be mixed with sialic acid or glucuronic acid on the same site of the membrane of the proteoliposome (usually the outside) (Cis-Inhibition test). When such compounds show interaction with the transport binding site a competive inhibition of the uptake of the radiolabeled sialic or glucuronic acid will be observed. When such compouds show inhibition, but in a non-competitive manner, they have interaction but not at the specific substrate binding site. If no inhibition is seen compounds do not show any interaction. To test whether compounds that show inhibition can actually be transported across the membrane a Trans-Stimulation test can be performed. In this test the compound to be tested must be preloaded in the proteoliposomes during their formation. If the compound is transported by the transporter it will be exchanged by the radioactive substrates sialic or glucuronic acid which are present on the other (outside) of the membrane of the proteoliposome. In this manner an increase of sialic or glucuronic acid uptake can be observed in preloaded proteoliposomes compared to uptake in not preloaded proteoliposomes.

Thus, the present invention provides for a method for identifying compounds that effect the transporter function of the protein according to the invention. The method comprises the steps of
a) introducing into a suitable host cell a nucleotide encoding the transporter according to the invention DNA
b) culturing the host cells under conditions to allow expression of the introduced DNA sequence;
c) bringing the host cell from step or a membrane fraction thereof into contact with target molecules and compounds potentially effecting the transporter function of the expressed protein encoded by DNA from step a;
d) establishing the transport efficiency of the expressed protein.

Optionally the expression product might be isolated and the transport measured on purified recombinant transporter.

As an alternative the protein might also be part of artificial membranes.

The present invention thus provides for a quick and economic method to screen for therapeutic agents for the prevention and/or treatment of diseases related to sialic acid or the transporter protein according to the invention. The method according to the invention furthermore provides for the selection of selective therapeutic agents discriminating between different transporters thus leading to a more effective therapeutic agent and/or diminishing of side effects. The method is especially suited to be used for the high throughput screening of numerous potential target compounds.

Compounds which activate or inhibit the transporter function may be employed in therapeutic treatments to activate or inhibit the transporter of the present invention.

The invention also provides for a method for the formulation of a pharmaceutical composition comprising mixing the activator or inhibitor compounds identified with a pharmaceutically acceptable carrier.

Pharmaceutical acceptable carriers are well known to those skilled in the art and include, for example, sterile salin, lactose, sucrose, calcium phosphate, gelatin, dextrin, agar, pectin, peanut oil, olive oil, sesame oil and water.

Furthermore the pharmaceutical composition according to the invention may comprise one or more stabilizers such as, for example, carbohydrates including sorbitol, mannitol, starch, sucrosedextrin and glucose, proteins such as albumin or casein, and buffers like alkaline phosphates. Methods for making preparations and intravenous admixtures are disclosed in Remingtons's Pharmaceutical Sciences, pp. 1463-1497 (16th ed. 1980, Mack Publ. Co of Easton, Pa, USA).

Thus, the inhibitor or activator compounds identified by using the transporter according to the invention are useful in the preparation of a pharmaceutical. The pharmaceutical is to be used in CNS or immune related disorders.

Also within the scope of the invention are antibodies, especially monoclonal antibodies raised against the transporter molecule according to the invention. Such antibodies can be used therapeutically to inhibit transporter function and diagnostically to detect transporter molecules. The antibodies can be prepared according to methods known in the art e.g. as described in EP488470.

The invention furthermore relates to the use of the transporter gene as part of a diagnostic assay for sialic acid transport defects related to mutations in the nucleic acid sequences encoding this transporter. Such mutations may e.g. be detected by using PCR (Saiki et al., Nature, 324, 163-166 (1986)) or specific hybridization. Also the relative levels of RNA can be determined using e.g. hybridization or quantitative PCR technology or DNA microarrays.

Also within the scope of the invention therefore are nucleic acid segments obtained from SEQ ID NO:2 which include a polymorphic site. Preferably these sites are located at positions 385, 803, 818, 1072, 1248, 1271 or 1382, most preferably at position 385.

The nucleic acid segments are preferably at least 10 contiguous nucleotides, preferably 10-100 nucleotides.

Another aspect of the invention relates to nucleotide segments having a nucleotide sequence capable of specifically hybridizing to the invariant proximal or invariant distal nucleotide sequence of a single nucleotide polymorphic site of SEQ ID NO:2, and being used to specifically detect the single nucleotide polymorphism site. Such nucleotide segments are especially useful in assays based on primer elongation methods such as e.g. PCR or NASBA.

The invention also relates to a method of analyzing a nucleic acid, comprising obtaining a nucleic acid from an individual; and determining a base occupying the polymorphic site of SEQ ID NO:2. preferably the sites at positions 385, 803, 818, 1072, 1248, 1271 or 1382, most preferably at position 385.

The presence and the levels of the transporters themselves can be assayed by immunological technologies such as radioimmuno assays, Western blots and ELISA using specific antibodies raised against the receptor. Such techniques for measuring RNA and protein levels are well known to the skilled artisan.

The determination of expression levels of different transporters in individual patients may lead to fine tuning of treatment protocols.

The following examples are illustrative for the invention and should in no way be interpreted as limiting the scope of the invention.

### Legends to the figures

### Figure 1

Northern blot analysis of sialin expression. The lanes correspond to the following tissues: lane 1, brain; 2, heart; 3, skeletal muscle; 4, colon; 5, thymus; 6, spleen; 7, kidney; 8, liver; 9, small intestine; 10, placenta; 11, lung; 12, peripheral blood leukocytes.

### Figure 2

Putative 2D model of human sialin. Membrane topology prediction algorithms suggest that sialin contains twelve transmembrane domains with the amino- and carboxy-termini facing towards the cytosol. Triangles indicate the six potential N-glycosylation sites. Small circles indicate amino acid residues. The mutation found in Salla disease is indicated in (a). A missense mutation R39C changes an arginine residue into a cysteine residue leading to a protein modification just before the first transmembrane domain. The different mutations found in infantile sialic acid storage disease, ISSD are indicated in (b). Mutations in patients (A-F) are indicated by boxes. For details of the mutations see Table 1. A: patient DR; B: patient AB; C: patient AZ; D: patient JG; E: patient RD; F: patient McI.

### Examples

### Example 1

### Identification of ESTs mapping at the SASD locus

Linkage analysis mapped the disease locus for Salla disease and for ISSD to the refined area on chromosome 6q14-q15 and a physical map was constructed over the disease locus, flanked by markers D6S280 and D6S1622. This area is covered by several overlapping genomic (PAC) clones. The Iluman Genemap 1998 provided by the international RII mapping consortium was screened for ESTs in the immediate vicinity of central marker D6S1596. Several overlapping clones from the genebank EST division were found, which showed a considerable similarity with a C. elegans hypothetical protein C38C10.2. This C. elegans protein has been classified within the ACS family of anion/cation symporters. This family contains a number of phosphate transporters as well as a bacterial glucuronic acid (hexuronate) transporter from E.coli (EXUT). Three Genbank clones 667681, 265499 and 209208 were used for subsequent studies. Phage library plating and screening conditions were as described (Sambrook, J., Fritsch, E.F. & Maniatis, T. *Molecular Cloning: A Laboratory Manual* (Cold Spring Harbor Laboratory Press, Cold Spring Harbor, New York, 1989). Human cDNA clones were isolated from human liver, brain and placenta Clontech cDNA libraries using probes derived from EST clones. cDNA inserts were isolated by PCR and sequenced. 5'RACE PCR was carried out using the Gibco BRL system according to the manufacturer's protocol.

### Example 2

### The sequence of a novel anion-cation symporter cDNA

The EST clones from example 1 hybridized to PAC202M22 covering the critical disease region and were thus considered to be part of a functional and positional candidate cDNA. By sequencing, it was found that these EST's represented only the 3'end of the cDNA, and therefore we used the same EST's as probes to screen a human fetal liver cDNA library (Clontech). Several overlapping positive clones were obtained. Screening more cDNA libraries (placenta and brain) with PCR amplified fragments of these clones, as well as rapid amplification of cDNA ends (RACE) resulted in a 2.5 kb sequence. This sequence included an ORF of 1485 bp, predicting a 495 amino acid protein sequence. Approximately 270 bp of 3' and 750 bp of 5' UTR have been sequenced. Upstream of the first ATG a stopcodon was found suggesting that the translation- initiating methionine has been obtained.

Searching for sequence homology using the BLAST program showed that this sequence has a significant homology with several members of the anion-cation symporter (ACS) family of transporters. The gene has been designated AST (anion and sugar transporter) (SEQ ID NO:2).

The predicted polypeptide encoded by AST gene contains twelve transmembrane domains, similar to that of most MFS members (SEQ ID NO:1). Extensive sequence homology was found with human brain and kidney Na+-phosphate symporters (Ni, B. et al. J. Neurochem. 66, 2227-2238 (1996); Chong, S.S. et al. Genomics 18, 355-359 (1993)) and with a H+-hexuronate symporter from E.coli (Hugouvieux-Cotte-Pattat, N. & Robert-Baudouy, J. of Bacteriol. 169, 1223-1231 (1987); Blattner, F.R. et al. Science 277, 1453-1474 (1997)). Sialin shows 37 %, 34 % and 16 % sequence identity with NaPbrain, NaPrenal and EcHex respectively. The similarity scores are 61 %, 57 % and 40 % respectively. Only the amino- and carboxyterminal regions do not show any significant homology.

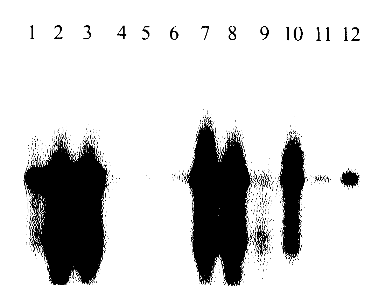

Northern analysis of human tissues showed two major transcripts of ≈4.5 and ≈3.5 kb, which are ubiquitously expressed but are more abundant in heart, kidney, skeletal muscle, liver, placenta and brain (Fig. 1).

A human 12-lane Multiple Tissue Northern (MTN) blot from Clontech was used. Hybridizations were performed using ExpressHyb (Clontech), according to the manufacturer's instructions. As probes we used either a 500 bp PCR product derived from the AST cDNA, or a specific 20-mer oligonucleotide 5'-CAGTGTGCTGCTCTGCTCGT-3' . Probes were 32P labeled and purified as described by the manufacturer.

### Example 3

### Mutations in Salla disease and ISSD patients

To detect mutations in the novel anion transporter gene (AST), primers were designed for RT-PCR analysis of cDNA from five confirmed typical Finnish Salla disease patients, from six earlier described patients with the severe infantile phenotype ISSD and from several controls. The five Salla patients shared the typical 1-3 haplotype of the closest markers known to be present in 91 percent of the Finnish Salla disease chromosomes. Most of the cell lines used for mutation analysis are known to have increased level of free sialic acid and were also used to demonstrate biochemically the transport defect.

The total cDNA was sequenced in four overlapping PCR's. RT-PCR reaction products were run on 1% agarose gels, isolated and sequenced by the ABI system in two directions. The results are summarized in Table 1.

**Table 1**

| AST mutations found in Salla disease and ISSD patients | | | |
|---|---|---|---|
| Nucleotide change | AA number/mutation | Protein consequence | Patients |
| **Salla Disease** | | | |
| 115 C > T | R39C | Arginine to cysteine in loop before first TM domain | Finnish Salla patients: LR, SE, SL, AA, PE homozygous |
| | | | |
| **ISSD** | | | |
| 533 del C | 78 | Frameshift resulting in 33 novel amino acids then premature truncation | French patient DR heterozygous |
| | | | |
| (1112-1259) del 148 bp | 371 | Frameshift resulting in 27 novel amino acids then premature truncation | French patient DR heterozygous |
| | | | |
| 548 A > G | H183R | Histidine to arginine in transmembrane domain 4 | Yugoslavian patient AZ heterozygous |
| | | | |
| 1001 C > G | P334R | Proline to arginine in transmembrane domain 8 | Yugoslavian patient AZ heterozygous |
| | | | |
| 978-979 ins 500 bp | 327 | Probably truncation | Italian patient AB homozygous |
| | | | |
| (802-816) del 15 bp | 268 del | Deletion of five amino acids [SSLRN] transmembrane domains 6 and 7 | French-Canad. patients JG and RD and English patient MeI apparently homozygous |
| Numbering of amino acids refers to the sialin protein sequence, numbering of nucleotides refers to the AST cDNA sequence, with the first nucleotide of the ATG initiation codon as nr.1. | | | |

Patient fibroblast or lymphoblast cell lines were established from Finnish Salla disease patients. Fibroblast strains from ISSD patients were obtained from previously described patients (see Table 1). Fibroblasts were cultured in Ham's F10 (Gibco BRL) supplemented with 15 % fetal calf serum and antibiotics. Lymphoblasts were cultured in RPMI 1640 (Gibco BRL) supplemented with 10 % fetal calf serum and antibiotics. Total RNA was isolated from control and patient cultured fibroblasts (or lymphoblasts) using RNAzol B (Campro Scientific) according to the manufacturer's protocol. Subsequently first strand cDNA was generated by the Superscript Preamplification system (Gibco BRL) using random hexamer priming. Gene specific primers were designed to facilitate 5'RACE and mutation analysis. cDNA was amplified by PCR using the Perkin Elmer 9600 Gene Amp PCR system. The PCR reaction amplification mixture (50 µl) contained dNTP's (25 mM each), forward and reverse primers (100 pmol), Taq DNA polymerase (5 U; Gibco BRL), 1x PCR buffer (Gibco BRL), 1% DMSO, 0.02 % Wl (Gibco BRL) and 2 mM MgCl₂. PCR conditions were 5 min. 95 °C; 40 cyles of 1 min. 95 °C, 1 min. 59 "C, 2 min. 72 "C; and 10 min.72 "C. PCR products were analysed on 1 % agarose gels, purified using QIAquick PCR purification kit (Qiagen) and sequenced using the ABI Prism Big Dye Terminator Cycle Sequencing kit (Applied Biosystems) and an Applied Biosystems ABI 377 fluorescent sequencer. The complete open reading frame and 5 prime and 3 prime UTR's were sequenced using 5 different overlapping primer pairs:

P1: f5'-GTGGCGAGTACACCTGCTCA-3'; r5'-GCAATGGGAGTGAACAGGGT-3';

P2: f5'-GATTTGGGATCCTTGGCACTG-3'; r5'-GTACCCACGGCACTGACTTC-3';

P3: f5'-GGAGCACAGCTTGGGACAGT-3'; r5'-CAGCAGCTTGACCAGACAGG-3';

P4: f5'-TGCCACTTTGGGCTATCGTAG-3'; r5'-GAACACGGTTTGCCATTCTTC-

3'; P5: f5'-CCTCCTGGGCATCACAAATAC-3'; r5'-
AACAGAACTGTCCTACTTCATG-3'.

Allele specific PCR was performed using as a 5'primer either 5'-CAGTGTGCTGCTCTGCTC-3' (wt) or 5'-CAGTGTGCTGCTCTGCTT-3' (mut) and as a 3'primer 5'-AGGCCAAAATTGCTAAGTTGT-3'. Cycling conditions were 95°C 11 min, 95°C 30 sec, 57°C 30 sec, 72°C 1 min for 35 cycles using MJResearch thermal cycler PTC-225 and AmplitaqGold (Roche).

### Example 4

### ISSD patients

Sequence analysis of six different ISSD patients of various ethnic origin revealed six different mutations (Table 1).

The ISSD patient of French origin (patient DR), was found to be a compound heterozygote with a 1 basepair deletion (533 del C) in one allele and a 148 basepairs deletion starting at position 1112 G till 1259 C in the other allele. Both deletions cause a frameshift and are predicted either to result in an unstable mRNA or in premature truncation of the protein.

The ISSD patient of Italian ancestry (patient AB), was homozygote for a ≈500 bp insertion at position 978 of the AST gene as observed by electrophoresis of PCR products and haplotype analysis. Subsequent sequencing identified the site of the insertion as indicated in Table 1.

The ISSD patient of Yugoslavian origin (patient AZ), showed two heterozygote missense mutations, 548A→G and 1001C→G in the AST sequence. The former leads to substitution of histidine with arginine (H183R) and the latter to substitution of proline with arginine (P334R), respectively. The effect of the H183R mutation cannot be predicted at present but the P334R mutation eliminates a highly conserved proline residue among ACS members and orthologues, and predictively leads to a major functional change. No other mutations were found in cDNA from this patient.

The French-Canadian ISSD patients (JG and RD) carried a 15 bp apparently homozygote deletion (de1802T-816T) leading to predicted deletion of five aminoacids SSLRN in the cytosolic loop between transmembrane domain 6 and 7.

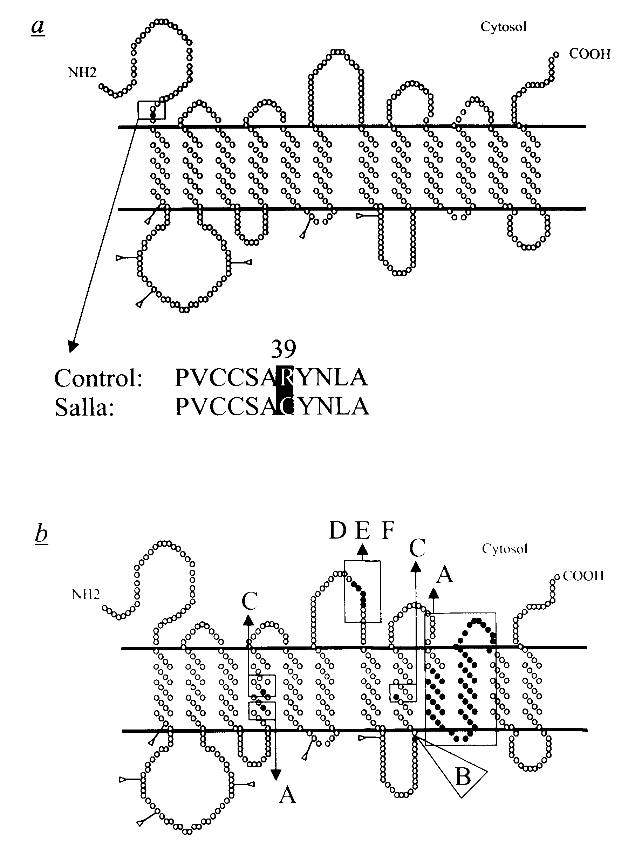

An English ISSD patient (McI) was found to carry this same homozygous 15 bp deletion. The mutations found in Salla disease and ISSD patients are indicated in a putative model of sialin (Fig. 2a and b).

## Claims

1. A polynucleotide encoding the amino acid sequence SEQ ID NO: 1.

2. The polynucleotide according to claim 1, said polynucleotide comprising the sequences SEQ ID NO:2 or the sequence extending from nucleotides 271-1755 from SEQ ID NO:2.

3. A recombinant expression vector comprising the DNA according to claims 1 or 2.

4. Protein encoded by the polynucleotide according to claims 1 or 2 or the expression vector according to claim 3.

5. A cell transfected with DNA according to claims 1 or 2 or the expression vector according to claim 3.

6. A cell according to claim 5 which is a stable transfected cell which expresses the protein according to claims 4.

7. Use of a DNA according to claims 1 or 2 or an expression vector according to claim 3, a cell according to claims 5 or 6 or a protein according to claim 4 in a screening assay for identification of new drugs.

8. A method for identifying compounds effecting the transporter function of the protein according to claim 4, said method comprising the steps of
a) introducing into a suitable host cell a DNA according to claims 1, 2 or an expression vector according to claims 3;
b) culturing the host cells under conditions to allow expression of the introduced DNA sequence;
c) optionally isolating the expression product;
d) bringing the host cell from step b, a membrane fraction thereof or the expression product from step c into contact with target molecules and compounds potentially effecting the transporter function of the expressed protein encoded by DNA from step a;
e) establishing the transport efficiency of the expressed protein.

9. The method according to claim 8 wherein the protein is integrated in an artificial membrane.

10. A method for the formulation of a pharmaceutical composition comprising the method of claims 8 or 9 and mixing the compound identified with a pharmaceutically acceptable carrier.

11. Use of a compound as identified in the method of claims 8 or 9 for the preparation of a pharmaceutical suitable as an activator or inhibitor of a sialic acid transporter protein.

12. A method of analyzing a nucleic acid, comprising obtaining a nucleic acid from an individual; and determining a base occupying the polymorphic site of SEQ ID NO:2.

13. A nucleic acid segment comprising at least 10 contiguous nucleotides including a polymorphic nucleotide of SEQ ID NO:2 or the complement thereof.

14. The nucleotide acid molecule of claim 13 wherein the segment is between 10 and 100 bases.

15. A nucleic acid segment having a nucleotide sequence capable of specifically hybridizing to the invariant proximal or invariant distal nucleotide sequence of a single nucleotide polymorphic site of SEQ ID NO;2, and being used to specifically detect the single nucleotide polymorphism site.

16. The nucleic acid segments of claims 13-15 wherein the polymorphic site is localized at position 385, 803, 818, 1072, 1248, 1271 or 1382.

17. The nucleic acid molecule of claims 16 wherein the polymorphic site is localized at position 385.
